# EUROPEAN PATENT APPLICATION

(11) **EP 4 180 510 A1**
(43) Date of publication of application: **17.05.2023**
(21) Application number: 21836774.6
(22) Date of filing: 23.06.2021
(51) Int. Cl.: C12M 1/00, C12M 3/04

(54) **CELL CULTURE SYSTEM**

(30) Priority: 07.07.2020 JP 2020116946
(71) Applicant: SHIMADZU CORPORATION, Kyoto-shi, Kyoto 604-8511 (JP)
(72) Inventor: HASHIMOTO, Toyoyuki, Kyoto-shi, Kyoto 604-8511 (JP); TAKUBO, Kenji, Kyoto-shi, Kyoto 604-8511 (JP); INOUE, Tsunehiro, Kyoto-shi, Kyoto 604-8511 (JP); YONEDA, Yasuko, Kyoto-shi, Kyoto 604-8511 (JP); OHKUBO, Tomoki, Kyoto-shi, Kyoto 604-8511 (JP)
(74) Representative: Kilian Kilian & Partner
(86) International application number: PCT/JP2021/023690
(87) International publication number: WO 2022/009672

(57) **Abstract**

A cell culture system includes: a first pump; and a first cell culture container and a second cell culture container. The first cell culture container has a first culture medium circulation flow path. The second cell culture container has a second culture medium circulation flow path independent of the first culture medium circulation flow path. The first culture medium circulation flow path and the second culture medium circulation flow path are fluidly connected to the first pump.

## Description

### TECHNICAL FIELD

The present invention relates to a cell culture system.

### BACKGROUND ART

PTL 1 (WO 2018/079793) discloses a system in which a cell culture container having intestinal epithelial cells seeded on a porous membrane is disposed in an anaerobic chamber and the intestinal epithelial cells and bacteria included in a culture medium are co-cultured.

### CITATION LIST

### PATENT LITERATURE

PTL 1: WO 2018/079793

### SUMMARY OF INVENTION

### TECHNICAL PROBLEM

In the conventional technology, however, sufficient consideration has not been made with regard to a design of a system suitable for performing a comparative analysis on cell states and culture medium components for the respective cell culture containers as a result of performing cell culture under various conditions.

The present invention provides a cell culture system suitable for an comparative analysis for respective cell culture containers.

### SOLUTION TO PROBLEM

A cell culture system of the present invention includes: a first pump; and a first cell culture container and a second cell culture container. The first cell culture container has a first culture medium circulation flow path. The second cell culture container has a second culture medium circulation flow path independent of the first culture medium circulation flow path. The first culture medium circulation flow path and the second culture medium circulation flow path are fluidly connected to the first pump.

### ADVANTAGEOUS EFFECTS OF INVENTION

According to the cell culture system of the present invention, a comparative analysis for the respective cell culture containers can be performed.

### BRIEF DESCRIPTION OF DRAWINGS

Fig. 1 is a perspective view of a cell culture system 100.
Fig. 2 is a cross sectional view of a base 10.
Fig. 3 is a cross sectional view of a cell culture container 20.
Fig. 4 is a perspective view of a rack 40 when attached to base 10.
Fig. 5 is a perspective view of a chamber apparatus 200.

### DESCRIPTION OF EMBODIMENTS

Embodiments of the present invention will be described in detail with reference to figures. In the figures described below, the same or corresponding portions are denoted by the same reference characters, and the same explanation will not be described repeatedly.

### (Configuration of Cell Culture System according to Embodiment)

Hereinafter, a configuration of a cell culture system (hereinafter, referred to as "cell culture system 100") according to an embodiment will be described.

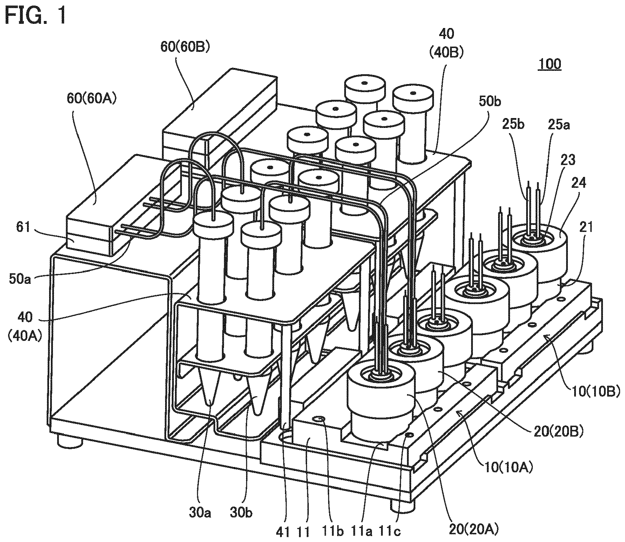

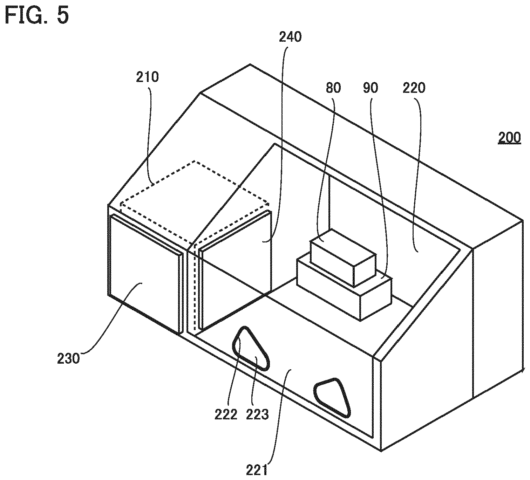

Fig. 1 is a perspective view of cell culture system 100. As shown in Fig. 1, cell culture system 100 has a base 10, a plurality of independent cell culture containers 20, a plurality of culture medium containers 30a and 30b, a rack 40, a plurality of tubes 50a and 50b, a pump 60, a plurality of lead wires 70a (not shown) and 70b (not shown). It should be noted that in Fig. 1, only two pairs of tubes 50a and 50b are shown. Cell culture system 100 further has a controller 80 (see Fig. 5), a transepithelial electrical resistance measurement apparatus 90 (see Fig. 5), and a chamber apparatus 200 (see Fig. 5). A plurality of bases 10, a plurality of racks 40, and a plurality of pumps 60 may be provided.

Each of the respective numbers of culture medium containers 30a and 30b, the respective numbers of tubes 50a and 50b, and the respective numbers of lead wires 70a and 70b is equal to the number of cell culture containers 20. In the example of Fig. 1, three cell culture containers 20 are provided for one base 10. However, in Fig. 1, only one pair of tubes 50a and 50b is shown.

Base 10 has a main body 11, a wiring board 12, a spring 13, and a ball 14. Main body 11 has an upper surface and a bottom surface. The bottom surface of main body 11 is opposite to the upper surface of main body 11. A recess 1 1a is formed in main body 11. In recess 11a, the upper surface of main body 11 is depressed toward the bottom surface side of main body 11. Recess 11a extends along a longitudinal direction of base 10 (main body 11).

Holes 11b and 11c are formed in main body 11. In each of holes 11b, the upper surface of main body 11 is depressed toward the bottom surface side of main body 11. Each of holes 11c is formed to communicate with an inner space of main body 11.

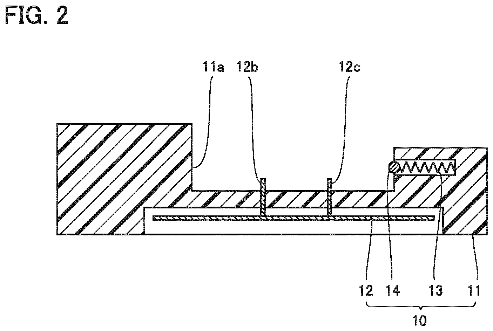

Fig. 2 is a cross sectional view of base 10. As shown in Fig. 2, wiring board 12 is disposed in the inner space of main body 11. Although not shown, wiring board 12 is provided with a wiring 12a. Wiring board 12 has a plurality of projection electrodes 12b and a plurality of projection electrodes 12c. Each of the number of projection electrodes 12b and the number of projection electrodes 12c is equal to the number of cell culture containers 20. One end of projection electrode 12b and one end of projection electrode 12c are electrically connected to wiring 12a. The other end of projection electrode 12b and the other end of projection electrode 12c project from the bottom surface of recess 11a.

Ball 14 is attached to a tip of spring 13. Spring 13 is embedded in base 10 such that ball 14 attached to the tip of spring 13 projects with respect to a side surface of recess 11a.

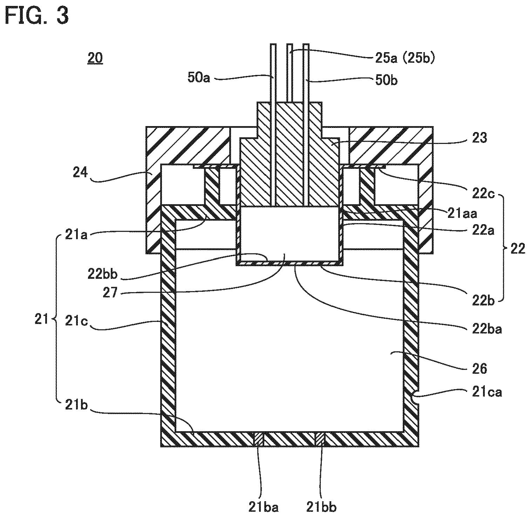

Fig. 3 is a cross sectional view of cell culture container 20. As shown in Fig. 3, cell culture container 20 has a container main body 21, a cell culture insert 22, a cover member 23, a cover member 24, and electrodes 25a and 25b.

A first culture medium 26 is stored in inside of container main body 21. Container main body 21 has an upper wall 21a, a bottom wall 21b, and a side wall 21c. Container main body 21 is preferably composed of a resin material.

An opening 21aa is formed in upper wall 21a. Opening 21aa extends through upper wall 21a along its thickness direction. It should be noted that upper wall 21a may be formed to be separated from bottom wall 21b and side wall 21c.

Bottom wall 21b faces upper wall 21a with a space being interposed therebetween. Electrodes 21ba and 21bb are embedded in bottom wall 21b. Electrodes 21ba and 21bb are electrically connected to first culture medium 26. Electrodes 21ba and 21bb are exposed from an outer surface of bottom wall 21b. Electrodes 21ba and 21bb are electrically connected to projection electrodes 12b and 12c respectively when container main body 21 is disposed in recess 11a.

Side wall 21c is contiguous to upper wall 21a and bottom wall 21b. A recess 21ca is formed in an outer surface of side wall 21c. Recess 21ca is formed at a position to face ball 14 when container main body 21 is disposed in recess 11a.

Cell culture insert 22 has a tubular portion 22a and an oxygen-permeable membrane 22b. The lower end side of tubular portion 22a is closed by membrane 22b. The upper end side of tubular portion 22a is closed by cover member 23. Cover member 23 is detachable from tubular portion 22a.

Second culture medium 27 is stored in inside of tubular portion 22a. A dissolved oxygen concentration of second culture medium 27 is lower than a dissolved oxygen concentration of first culture medium 26. That is, first culture medium 26 is an aerobic culture medium, and second culture medium 27 is an anaerobic culture medium. Second culture medium 27 includes, for example, anaerobic bacteria.

Tubular portion 22a is inserted in opening 21aa such that the lower end side of tubular portion 22a is located in the inside of container main body 21. In this way, cell culture insert 22 is attached to container main body 21. Cell culture insert 22 is detachable from container main body 21.

Membrane 22b is, for example, a track-etched membrane composed of polycarbonate. Membrane 22b has a first main surface 22ba and a second main surface 22bb. First main surface 22ba faces the inside of container main body 21. Second main surface 22bb faces the inside of tubular portion 22a. Second main surface 22bb is opposite to first main surface 22ba.

Cells are cultured on second main surface 22bb. These cells are, for example, intestinal epithelial cells that form a tight junction on second main surface 22bb. Specific examples of such cells include Caco-2 cells. Oxygen in first culture medium 26 is supplied to the cells through membrane 22b.

Cover member 24 is detachably attached to container main body 21. In this way, cell culture insert 22 is prevented from falling off from container main body 21. It should be noted that an opening is formed in cover member 24 and the upper surface of cover member 23 is exposed from the opening.

Electrodes 25a and 25b are inserted in cover member 23. One end of electrode 25a and one end of electrode 25b are electrically connected to second culture medium 27. The other end of electrode 25a and the other end of electrode 25b are exposed from cover member 23.

Cell culture container 20 is detachably attached to base 10. More specifically, container main body 21 is disposed in recess 11a to thereby attach cell culture container 20 to base 10. With this attachment, electrodes 21ba and 21bb are electrically connected to projection electrodes 12b and 12c, respectively.

Ball 14 is in contact with recess 21ca in the state in which cell culture container 20 is attached to base 10. Spring 13 generates biasing force toward side wall 21c through ball 14. Thus, positional deviation of cell culture container 20 (container main body 21) in recess 11a is suppressed.

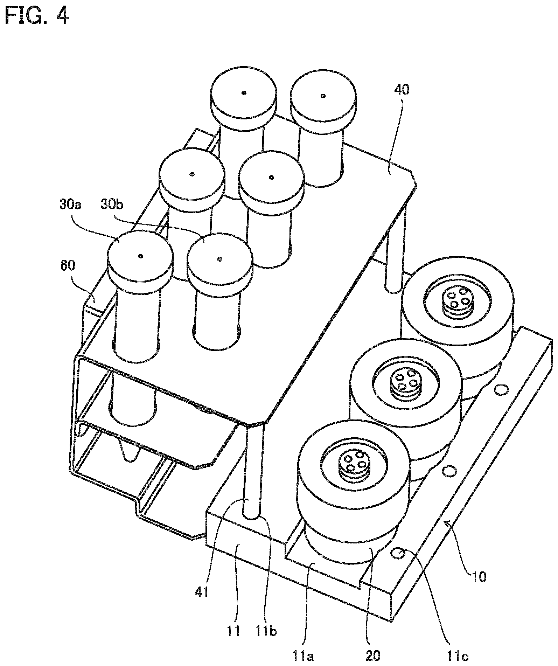

Culture medium containers 30a and 30b are supported by rack 40. Fig. 4 is a perspective view of rack 40 when attached to base 10. In Fig. 4, tubes 50a and 50b are not shown. As shown in Fig. 4, rack 40 is detachably attached to base 10. More specifically, rack 40 has supporting pillars 41. Rack 40 is attached to base 10 by inserting supporting pillars 41 into holes 11b. Rack 40 is composed of a magnetic material (soft magnetic material).

Second culture medium 27 is stored in culture medium container 30a. One end of tube 50a is inserted in cover member 23. Thus, tube 50a and the inside of tubular portion 22a are connected to each other. The other end of tube 50a is connected to culture medium container 30a. One end of tube 50b is inserted in cover member 23. Thus, tube 50b and the inside of tubular portion 22a are connected to each other.

Pump 60 is attached to tube 50a. Pump 60 delivers second culture medium 27 stored in culture medium container 30a to the inside of tubular portion 22a via tube 50a. Pump 60 is attached to tube 50b. Pump 60 delivers second culture medium 27 stored in tubular portion 22a to culture medium container 30b via tube 50b. That is, by operating pump 60, second culture medium 27 stored in tubular portion 22a is replaced. In cell culture system 100, since the supply of second culture medium 27 from culture medium container 30a to cell culture container 20 and the collection of second culture medium 27 from cell culture container 20 to culture medium container 30b are performed by one pump 60, a deviation between an amount of supply of the culture medium and an amount of collection of the culture medium due to an difference between individual pumps can be reduced.

Pump 60 is a pump that can perform the above-described delivering without making contact with second culture medium 27. Pump 60 is, for example, a tube pump. However, pump 60 is not limited thereto. Pump 60 may be, for example, a syringe pump.

Pump 60 has a magnet 61. Since rack 40 is composed of a magnetic material, pump 60 can be attached to rack 40 by magnet 61 (see Fig. 4).

Tube 50a is detachable from cover member 23 and culture medium container 30a. Tube 50b is detachable from cover member 23 and culture medium container 30b. Pump 60 is detachable from tube 50a.

It should be noted that tube 50a connected to each of the plurality of cell culture containers 20 is preferably connected to one pump 60. Thus, in cell culture system 100, the supply and collection of second culture medium 27 to and from one (hereinafter, referred to as "cell culture container 20A") of the plurality of cell culture containers 20 and the supply and collection of second culture medium 27 to and from the other one (hereinafter, referred to as "cell culture container 20B") of the plurality of cell culture containers 20 are performed by one pump 60, so that flow rate control can be performed for cell culture container 20A and cell culture container 20B in the same manner. A culture condition (for example, a seeding amount of cells, a type of bacteria, or the like) other than the flow rate may be the same between cell culture container 20A and cell culture container 20B. However, the culture condition other than the flow rate may be different between cell culture container 20A and cell culture container 20B.

One of the plurality of bases 10 is referred to as "base 10A". The other one of the plurality of bases 10 is referred to as "base 10B". Rack 40 attached to base 10A is referred to as "rack 40A". Rack 40 attached to base 10B is referred to as "rack 40B". Pump 60 attached to rack 40A is referred to as "pump 60A". Pump 60 attached to rack 40B is referred to as "pump 60B". Flow control for pump 60A may be different from flow control for pump B. A culture condition (for example, a seeding amount of cells, a type of bacteria, or the like) other than the flow rate may be different between cell culture container 20 located on base 10A and cell culture container 20 located on base 10B. However, the culture condition (for example, the seeding amount of cells, the type of bacteria, or the like) other than the flow rate may be the same between cell culture container 20 located on base 10A and cell culture container 20 located on base 10B.

One end of lead wire 70a and one end of lead wire 70b are electrically connected to wiring board 12 (wiring 12a) through holes 11c. One end of lead wire 70a and one end of lead wire 70b are preferably detachable from wiring board 12. The other end of lead wire 70a and the other end of lead wire 70b are detachably electrically connected to electrodes 25a and 25b by IC clips, for example.

Controller 80 controls pump 60. More specifically, controller 80 includes a microcontroller. The microcontroller generates a control signal corresponding to a content of input from an operation button or the like of controller 80, and transmits the control signal to pump 60 via a wiring (not shown). In this way, pump 60 is controlled.

Transepithelial electrical resistance measurement apparatus 90 is connected to wiring board 12 via a wiring (not shown), and measures electrical resistance values between electrodes 21ba and 21bb and electrodes 25a and 25b via the wiring, wiring board 12, lead wire 70a, and lead wire 70b. This measurement is performed, for example, by a four-terminal method.

It should be noted that since there are the plurality of electrodes 21ba, the plurality of electrodes 21bb, the plurality of electrodes 25a, and the plurality of electrodes 25b, a plurality of signals are output from wiring board 12. The plurality of outputs are input to one distributor. The distributor selects one of the plurality of outputs in a time-division manner and inputs the selected output to transepithelial electrical resistance measurement apparatus 90. Thus, it is unnecessary to prepare a plurality of transepithelial electrical resistance measurement apparatuses 90.

The electrical resistance values between electrodes 21ba and 21bb and electrodes 25a and 25b are varied between a case where the cells cultured on second main surface 22bb form a tight junction and a case where the cells cultured on the second main surface 22bb do not form a tight junction. Therefore, by the measurement of the electrical resistance values, it is possible to determine whether or not the cells cultured on second main surface 22bb form a tight junction.

Cell culture system 100 may further has an oxygen sensor (not shown). The oxygen sensor is disposed on a path of tube 50b, and is configured to detect a dissolved oxygen concentration of second culture medium 27 flowing through tube 50b. Thus, cell culture system 100 can monitor a growth state of bacteria during co-culture.

Fig. 5 is a perspective view of chamber apparatus 200. As shown in Fig. 5, chamber apparatus 200 has an air lock 210 and an anaerobic chamber 220. Air lock 210 is partitioned from the outside of chamber apparatus 200 by an outer door 230. Air lock 210 is partitioned from anaerobic chamber 220 by an inner door 240. An anaerobic environment (environment with a low oxygen concentration) is maintained in anaerobic chamber 220.

Anaerobic chamber 220 has a front panel 221. Arm ports 222 are formed in front panel 221. Each of arm ports 222 extends through front panel 221 and communicates with an inner space of anaerobic chamber 220. Various operations can be performed in anaerobic chamber 220 by inserting a hand into a sleeve (not shown) or glove (not shown) attached to arm port 222. Arm port 222 is closed by an arm port door 223.

In order to place an object in anaerobic chamber 220, first, outer door 230 is opened, the object is placed in air lock 210, and outer door 230 is closed. Second, the inside of air lock 210 is made anaerobic. Third, after completion of making the inside of air lock 210 anaerobic, inner door 240 is opened to move the object in air lock 210 into anaerobic chamber 220. In order to remove the object from anaerobic chamber 220, an operation reverse to the foregoing operation is performed.

Base 10, cell culture container 20, culture medium containers 30a and 30b, rack 40, tubes 50a and 50b, pump 60, lead wires 70a and 70b, controller 80, and transepithelial electrical resistance measurement apparatus 90 are disposed in anaerobic chamber 220.

Controller 80 and transepithelial electrical resistance measurement apparatus 90 are permanently disposed in an anaerobic chamber 220. However, base 10, cell culture container 20, culture medium containers 30a and 30b, rack 40, tube 50a and tube 50b, pump 60, and lead wires 70a and 70b can be removed from anaerobic chamber 220.

It should be noted that by analyzing second culture medium 27 in culture medium container 30b and first culture medium 26 in container main body 21 both removed from anaerobic chamber 220, dynamics of metabolism and absorption during co-culture can be analyzed.

### (Effects of Cell Culture System according to Embodiment)

Effects of cell culture system 100 will be described below.

Since cell culture system 100 has the plurality of cell culture containers 20 independent of each other, a comparative analysis can be performed for respective cell culture containers 20. In cell culture system 100, the plurality of independent cell culture containers 20 are detachably attached to one base 10. Therefore, according to cell culture system 100, the plurality of independent cell culture containers 20 can be carried together with base 10, so that the plurality of independent cell culture containers 20 can be readily removed from anaerobic chamber 220.

Cell culture system 100 has culture medium containers 30a and 30b, tubes 50a and 50b, and pump 60. Therefore, according to cell culture system 100, the cells and bacteria can be co-cultured while replacing second culture medium 27 stored in tubular portion 22a.

In cell culture system 100, rack 40 that supports the plurality of culture medium containers 30a and the plurality of culture medium containers 30b can be detachably attached to base 10. Further, in cell culture system 100, pump 60 can be attached to rack 40 by magnet 61. Therefore, according to cell culture system 100, base 10, cell culture containers 20, culture medium containers 30a and 30b, rack 40, tubes 50a and 50b, and pump 60 can be collectively removed from anaerobic chamber 220.

Each of container main body 21, cell culture insert 22, culture medium containers 30a and 30b, tubes 50a and 50b is in contact with a culture medium. Therefore, these components are preferably discarded after performing the co-culture using cell culture system 100 and are preferably replaced with new components. However, if these components are not detachable, these components must be reused after being subjected to a sterilization process with an autoclave or the like, thus resulting in decreased efficiency of experiment.

In cell culture system 100, cell culture insert 22 is detachable from container main body 21 and cover member 23 is detachable from cell culture insert 22. Further, in cell culture system 100, tube 50a is detachable from cover member 23 and culture medium container 30a, and tube 50b is detachable from cover member 23 and culture medium container 30b. Further, in cell culture system 100, pump 60 is detachable from tube 50a.

Thus, according to cell culture system 100, the components to be sterilized and reused and the components to be discarded can be separated, with the result that efficiency of the sterilization process can be increased and thus the efficiency of experiment can be increased.

In cell culture system 100, since spring 13 generates biasing force toward side wall 21c through ball 14, positional deviation of cell culture container 20 in recess 11a is suppressed. Thus, even when a light-weight material (resin material or the like) is used for container main body 21 and therefore cell culture container 20 is likely to be shaken, stable co-culture can be performed.

In cell culture system 100, since the electrical resistance values between electrodes 21ba and 21bb and electrodes 25a and 25b are measured by transepithelial electrical resistance measurement apparatus 90, the co-culture can be proceeded while monitoring states of the cells cultured on second main surface 22bb.

Although the embodiments of the present invention have been illustrated, the embodiments described above can be modified in various manners. Further, the scope of the present invention is not limited to the above-described embodiments. The scope of the present invention is defined by the terms of the claims, and is intended to include any modifications within the scope and meaning equivalent to the terms of the claims.

### REFERENCE SIGNS LIST

10, 10A, 10B: base; 11: main body; 11a: recess; 11b, 11c: hole; 12: wiring board; 12a: wiring; 12b, 12c: projection electrode; 13: spring; 14: ball; 20, 20A, 20B: cell culture container; 21: container main body; 21a: upper wall; 21aa: opening; 21b: bottom wall; 21ba: electrode; 21bb: electrode; 21c: side wall; 21ca: recess; 22: cell culture insert; 22a: tubular portion; 22b: membrane; 22ba: first main surface; 22bb: second main surface; 23, 24: cover member; 25a, 25b: electrode; 26: first culture medium; 27: second culture medium; 30a, 30b: culture medium container; 40: rack; 40A, 40B: rack; 41: supporting pillar; 50a, 50b: tube; 60, 60A, 60B: pump; 61: magnet; 70a, 70b: lead wire; 80: controller; 90: transepithelial electrical resistance measurement apparatus; 100: cell culture system; 200: chamber apparatus; 210: air lock; 220: anaerobic chamber; 221: front panel; 222: arm port; 223: arm port door; 230: outer door; 240: inner door.

## Claims

1. A cell culture system comprising:
a first pump; and
a first cell culture container and a second cell culture container, wherein
the first cell culture container has a first culture medium circulation flow path,
the second cell culture container has a second culture medium circulation flow path independent of the first culture medium circulation flow path, and
the first culture medium circulation flow path and the second culture medium circulation flow path are fluidly connected to the first pump.

2. The cell culture system according to claim 1, further comprising a first base, wherein
the first cell culture container and the second cell culture container are detachably attached to the first base.

3. The cell culture system according to claim 1 or 2, further comprising:
a second pump; and
a third cell culture container and a fourth cell culture container, wherein
the third cell culture container has a third culture medium circulation flow path,
the fourth cell culture container has a fourth culture medium circulation flow path independent of the third culture medium circulation flow path, and
the third culture medium circulation flow path and the fourth culture medium circulation flow path are fluidly connected to the second pump.

4. The cell culture system according to claim 3, further comprising a second base, wherein
the third cell culture container and the fourth cell culture container are detachably attached to the second base.

5. The cell culture system according to claim 2, wherein the first culture medium circulation flow path is fluidly connected to a culture medium supply container and a culture medium collection container,
the cell culture system further comprising a rack that supports the culture medium supply container and the culture medium collection container, wherein
the rack is detachably attached to the first base.

6. The cell culture system according to claim 2, wherein the first cell culture container includes an oxygen-permeable membrane disposed in the first cell culture container, the membrane partitioning a first storage portion in which a first culture medium is stored and a second storage portion in which a second culture medium having a dissolved oxygen concentration lower than a dissolved oxygen concentration of the first culture medium is stored.

7. The cell culture system according to claim 6, wherein
the first cell culture container has
a container main body,
a cell culture insert including a tubular portion, the cell culture insert having a bottom surface on which the membrane is disposed, and
a cover member that seals the container main body,
the container main body includes an upper wall provided with an opening, a bottom wall facing the upper wall with a space being interposed between the bottom wall and the upper wall, and a side wall contiguous to the upper wall and the bottom wall,
a first end of the tubular portion is closed by the membrane,
a second end of the tubular portion is closed by the cover member,
the tubular portion is inserted in the opening of the upper wall such that the first end of the tubular portion is located in inside of the container main body,
the first culture medium is stored in the inside of the container main body, and
the second culture medium is stored in inside of the tubular portion.

8. The cell culture system according to claim 7, further comprising:
a first tube and a second tube; and
a culture medium supply container and a culture medium collection container, wherein
a first end of the first tube and a first end of the second tube are inserted in the cover member of the first cell culture container and are connected to the inside of the tubular portion,
a second end of the first tube and a second end of the second tube are respectively connected to the culture medium supply container and the culture medium collection container, and
the first pump is configured to deliver the second culture medium from the culture medium supply container to the inside of the tubular portion of the first cell culture container via the first tube, and to deliver the second culture medium from the inside of the tubular portion of the first cell culture container to the culture medium collection container via the second tube.

9. The cell culture system according to claim 8, wherein
the cell culture insert is detachable from the container main body,
the cover member is detachable from the cell culture insert,
the first tube and the second tube are respectively detachable from the culture medium supply container and the culture medium collection container,
the first tube and the second tube are each detachable from the first pump, and
the first tube and the second tube are detachable from the cover member of the first cell culture container.

10. The cell culture system according to claim 8 or 9, further comprising: a rack that supports the culture medium supply container and the culture medium collection container, the rack being detachably attached to the base, wherein
the first pump has a magnet, and
the rack is composed of a magnetic material.

11. The cell culture system according to any one of claims 7 to 10, wherein
the first base is provided with a recess in which the first cell culture container and the second cell culture container are disposed, and
a spring is embedded in the first base to generate biasing force toward the side wall of each of the first cell culture container and the second cell culture container disposed in the recess.

12. The cell culture system according to any one of claims 7 to 11, further comprising a first lead wire, wherein
the first base has a wiring board,
the first cell culture container has a first electrode that is embedded in the bottom wall and that is electrically connected to the first culture medium, and a second electrode that extends through the cover member and that is electrically connected to the second culture medium,
the first electrode of the first cell culture container is electrically connected to the wiring board due to the first cell culture container being attached to the first base,
a first end of the first lead wire is electrically connected to the second electrode of the first cell culture container, and
a second end of the first lead wire is electrically connected to the wiring board.

13. The cell culture system according to claim 12, further comprising:
an anaerobic chamber;
a controller that controls the first pump; and
a transepithelial electrical resistance measurement apparatus that is electrically connected to the wiring board and that measures an electrical resistance value between the first electrode of the first cell culture container and the second electrode of the first cell culture container, wherein
the controller and the transepithelial electrical resistance measurement apparatus are permanently disposed in the anaerobic chamber.
